Europäisches Patentamt
European Patent Office
Office européen des brevets

(19) (11) Veröffentlichungsnummer: **0 357 043 A2**

# (12) EUROPÄISCHE PATENTANMELDUNG

(21) Anmeldenummer: 89116017.8

(22) Anmeldetag: 30.08.89

(51) Int. Cl.5: C07D 487/04 , C07D 495/14 , A61K 31/55 , //(C07D487/04, 243:00,235:00),(C07D495/14, 333:00,243:00,235:00)

Patentansprüche für folgende Vertragsstaaten: ES + GR

(30) Priorität: 31.08.88 CH 3237/88
05.06.89 CH 2108/89

(43) Veröffentlichungstag der Anmeldung: 07.03.90 Patentblatt 90/10

(84) Benannte Vertragsstaaten: AT BE CH DE ES FR GB GR IT LI LU NL SE

(71) Anmelder: F. HOFFMANN-LA ROCHE AG
Postfach 3255
CH-4002 Basel(CH)

(72) Erfinder: Hunkeler, Walter, Dr.
Im Stigler 32
CH-4312 Magden(CH)
Erfinder: Kyburz, Emilio, Dr.
Unterer Rebbergweg 127
CH-4153 Reinach(CH)
Erfinder: Meier, Marc
75 Av. du Gen. de Gaulle
F-68300 Village-Neuf(FR)

(74) Vertreter: Lederer, Franz, Dr. et al
Patentanwalt Dr. Franz Lederer
Lucile-Grahn-Strasse 22
D-8000 München 80(DE)

## (54) Imidazodiazepin-Derivate.

(57) Die neuen Imidazodiazepin-Derivate der allgemeinen Formel

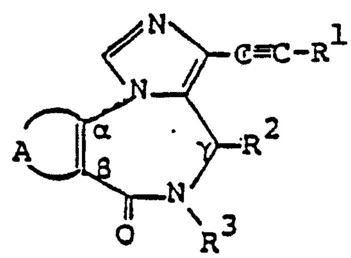

I

worin A zusammen mit den beiden mit α und β bezeichneten Kohlenstoffatomen eine der Gruppen

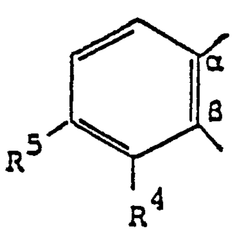 (a)

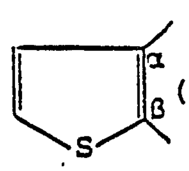 (b) und

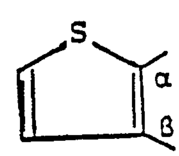 (c),

EP 0 357 043 A2

Xerox Copy Centre

$R^1$ eine gegebenenfalls durch Hydroxy, Oxo, Aryl oder die Gruppe -OR, -SR oder -OCOR substituierte, partiell ungesättigte niedere Kohlenwasserstoffgruppe oder eine durch Aryl oder die Gruppe -OR′, -SR′ oder -OCOR′ substituierte niedere Alkylgruppe, R und R′ je Aryl oder eine gegebenenfalls durch Aryl oder niederes Alkoxy substituierte, gesättigte oder partiell ungesättigte $C_{1-18}$-Kohlenwasserstoff- gruppe, $R^2$ Wasserstoff und $R^3$ niederes Alkyl oder $R^2$ und $R^3$ zusammen Dimethylen oder Trimethylen und $R^4$ und $R^5$ je Wasserstoff, Halogen, Trifluormethyl, Cyano, Nitro oder niederes Alkyl bedeuten, wobei die Gruppe -OR′ eine von niederem Alkoxy verschiedene Bedeutung hat und wobei die Verbindungen der Formel I bezüglich des mit $\gamma$ bezeichneten Kohlenstoffatoms die (S)- oder (R,S)-Konfiguration aufweisen, wenn $R^2$ und $R^3$ zusammen Dimethylen oder Trimethylen bedeuten,
können bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten verwendet werden.

## Imidazodiazepin-Derivate

Die vorliegende Erfindung betrifft Imidazodiazepin-Derivate. Im speziellen betrifft sie Imidazodiazepin-Derivate der allgemeinen Formel

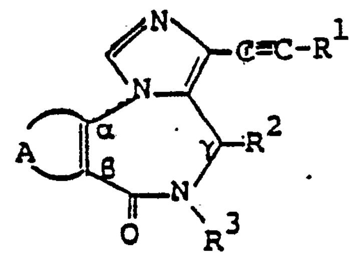

I

worin A zusammen mit den beiden mit α und β bezeichneten Kohlenstoffatomen eine der Gruppen

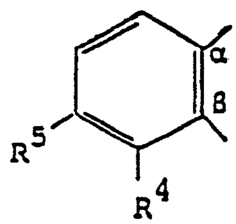 (a)

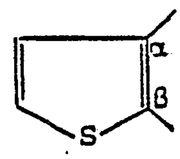 (b) und

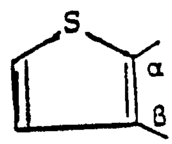 (c),

$R^1$ eine gegebenenfalls durch Hydroxy, Oxo, Aryl oder die Gruppe -OR, -SR oder -OCOR substituierte, partiell ungesättigte niedere Kohlenwasserstoffgruppe oder eine durch Aryl oder die Gruppe -OR′, -SR′ oder -OCOR′ substituierte niedere Alkylgruppe, R und R′ je Aryl oder eine gegebenenfalls durch Aryl oder niederes Alkoxy substituierte, gesättigte oder partiell ungesättigte $C_{1-18}$-Kohlenwasserstoff- gruppe, $R^2$ Wasserstoff und $R^3$ niederes Alkyl oder $R^2$ und $R^3$ zusammen Dimethylen oder Trimethylen und $R^4$ und $R^5$ je Wasserstoff, Halogen, Trifluormethyl, Cyano, Nitro oder niederes Alkyl bedeuten, wobei die Gruppe -OR′ eine von niederem Alkoxy verschiedene Bedeutung hat und wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen oder Trimethylen bedeuten.

Diese Verbindungen sind neu und zeichnen sich durch wertvolle pharmakodynamische Eigenschaften aus.

Gegenstand der vorliegenden Erfindung sind die Verbindungen der obigen Formel I als solche und als therapeutische Wirkstoffe, Verfahren zu deren Herstellung, Arzneimittel, enthaltend eine Verbindung der Formel I und einen therapeutisch inerten Träger, die Herstellung solcher Arzneimittel und die Verwendung von Verbindungen der Formel I bei der Bekämpfung oder Verhütung von Krankheiten (insbesondere bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten) bzw. die Verwendung von Verbindungen der Formel I zur Herstellung von Arzneimitteln, insbesondere von Arzneimitteln zur Anwendung bei den soeben erwähnten Indikationen.

Der Ausdruck "nieder" wird verwendet, um Reste und Verbindungen mit bis zu 7, vorzugsweise bis zu 4 Kohlenstoffatomen zu bezeichnen. Der Ausdruck "Alkyl" bezeichnet geradkettige oder verzweigte gesättigte Kohlenwasserstoffreste wie Methyl, Aethyl, n-Propyl, Isopropyl, n-Butyl, Isobutyl, s-Butyl, t-Butyl und dergleichen. Der Ausdruck "Alkoxy" bezeichnet über ein Sauerstoffatom gebundene Alkylreste im Sinne der vorstehenden Definition des Ausdruckes "Alkyl". Der Ausdruck "Alkenyl" bezeichnet geradkettige oder verzweigte Kohlenwasserstoffreste, welche mindestens eine olefinische Doppelbindung enthalten, wie cis- und trans-2-Buten-2-yl und 1-Buten-3-yl. Der Ausdruck "Aryl" bezeichnet vorzugsweise monocyclische aromatische Kohlenwasserstoffreste, welche vorzugsweise unsubstituiert oder durch niederes Alkyl, niederes Alkoxy und/oder Halogen substituiert sind. Falls nichts anderes angegeben ist, bezeichnet der Ausdruck "Halogen" die vier Halogene Fluor, Chlor, Brom und Jod.

Der Ausdruck "Kohlenwasserstoffgruppe" bezeichnet offenkettige und cyclische Gruppen und Kombinationen davon. Die offenkettigen Gruppen können geradkettig oder verzweigt sein. Beispiele für gesättigte niedere Kohlenwasserstoffgruppen sind: Methyl, Aethyl, i-Propyl, t-Butyl, 3-Pentyl, 1-Nonyl, Cyclopropyl, Cyclopentyl, Cyclohexyl, Cyclopropylmethyl, Cyclohexylmethyl und 1-Cyclopropyläthyl. Beispiele für ungesättigte Kohlenwasserstoffgruppen sind beispielsweise die oben erwähnten Alkenylgruppen, insbesondere niedere Alkenylgruppen, oder Kohlenwasserstoffreste, welche mindestens eine acetylenische Dreifachbindung enthalten, d.h. Alkinyl-, insbesondere niedere Alkinylgruppen, wie 1-Propin-3-yl.

$R^1$ bedeutet vorzugsweise eine gegebenenfalls durch Hydroxy, Oxo, Aryl oder die Gruppe -OR, -SR oder -OCOR substituierte niedere Alkenylgruppe oder eine durch Aryl oder die Gruppe -OR′, -SR′ oder -OCOR′ substituierte niedere Alkylgruppe, insbesondere eine durch Hydroxy oder die Gruppe -OR, -SR oder -OCOR substituierte niedere Alkenylgruppe oder eine durch die Gruppe -OR′, -SR′ oder -OCOR′ substituierte niedere Alkylgruppe. R und R′ bedeuten vorzugsweise je Aryl, Aryl-niederes Alkyl, niederes Alkoxy-niederes Alkyl, niederes Alkyl, $C_{3-7}$-Cycloalkyl oder $C_{3-7}$-Cycloalkyl-niederes Alkyl.

Wenn $R^2$ Wasserstoff und $R^3$ niederes Alkyl bedeuten, dann steht $R^3$ vorzugsweise für Methyl. Wenn $R^2$ und $R^3$ zusammen Dimethylen oder Trimethylen bedeuten, dann weist das mit $\gamma$ bezeichnete Kohlenstoffatom vorzugsweise die (S)-Konfiguration auf.

Wenn A einen Rest der Formel (a) bedeutet, dann bedeutet vorzugsweise eines von $R^4$ und $R^5$ Wasserstoff und das andere Wasserstoff oder Halogen; dabei bedeuten beispielsweise $R^4$ und $R^5$ beide Wasserstoff oder $R^4$ Wasserstoff und $R^5$ Fluor oder $R^4$ Chlor oder Brom und $R^5$ Wasserstoff.

Im Rahmen der vorliegenden Erfindung bevorzugte Verbindungen der Formel I sind:

7-Chlor-3-[3-(cyclopropylmethoxy)-1-propinyl]-4,5-dihydro-5-methyl-6H-imidazo[1,5-a][1,4]benzodiazepin-6-on,

3-(7-Chlor-5,6-dihydro-5-methyl-6-oxo-4H-imidazo[1,5-a][1,4]benzodiazepin-3-yl)-2-propinyl acetat,

7-Chlor-3-[3-(cyclopropylmethoxy)-3-methyl-1-butinyl]-5-methyl-6H-imidazo[1,5-a][1,4]benzodiazepin-6-on,

7-Chlor-4,5-dihydro-3-[(Z)-5-hydroxy-3-methyl-3-penten-1-inyl]-5-methyl-6H-imidazo[1,5-a][1,4]-benzodiazepin-6-on,

7-Chlor-4,5-dihydro-3-(3-hydroxy-3-methyl-4-penten-1-inyl)-5-methyl-6H-imidazo[1,5-a][1,4]benzodiazepin-6-on und

7-Chlor-3-[(E)-5-hydroxy-3-methyl-3-penten-1-inyl]-5-methyl-6H-imidazo[1,5-a][1,4]benzodiazepin-6-on.

Die Verbindungen der Formel I können erfindungsgemäss dadurch hergestellt werden, dass man

a) eine Verbindung der allgemeinen Formel

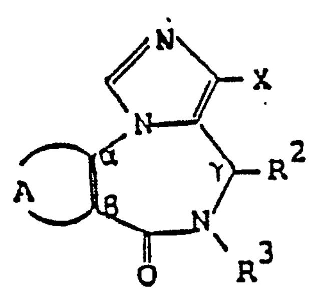

II

worin A, $R^2$ und $R^3$ obige Bedeutung besitzen, und X Brom oder Jod bedeutet, wobei aber im Falle, dass A einen Rest der Formel (a) und $R^4$ und/oder $R^5$ Halogen bedeuten, dieses Halogen Fluor oder Chlor ist, wenn X Brom bedeutet, bzw. Fluor, Chlor oder Brom ist, wenn X Jod bedeutet,

mit einer Verbindung der allgmeinen Formel

$HC \equiv C-R^1$ III

worin $R^1$ obige Bedeutung besitzt,

umsetzt; oder

b) eine Verbindung der Formel I, worin $R^1$ eine durch Hydroxy substituierte, partiell ungesättigte niedere Kohlenwasserstoffgruppe bedeutet, oder eine Verbindung der allgemeinen Formel

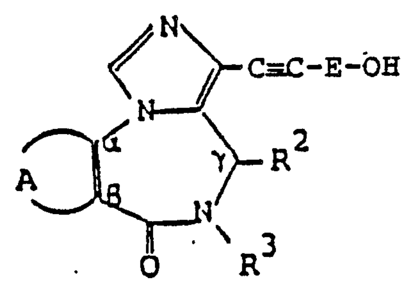

IV

worin E niederes Alkylen bedeutet, und $R^2$, $R^3$ und A obige Bedeutung besitzen, mit einem die Gruppe -OR oder -OCOR, bzw. -OR′ oder -OCOR′ liefernden Mittel veräthert bzw. acyliert.

Die Umsetzung von Verbindungen der Formeln II und III gemäss Aspekt a) des erfindungsgemässen Verfahrens erfolgt in Gegenwart eines Palladium(II)-Salzes, wie Palladiumchlorid oder Palladiumacetat, eines Organophosphins, wie Triphenylphosphin, von Kupfer(I)-jodid und eines sekundären oder tertiären Amins, wie Diäthylamin oder Triäthylamin. Anstatt eines Palladium(II)-salzes und eines Organophosphins kann auch ein geeigneter entsprechender Komplex verwendet werden, wie z.B. Bis(triphenylphosphin)palladium(II)-dichlorid. Als Lösungsmittel kann das erwähnte sekundäre oder tertiäre Amin selbst, ein halogenierter Kohlenwasserstoff, wie Methylenchlorid, N,N-Dimethylformamid oder eine Mischung davon verwendet werden. Die Reaktion erfolgt bei Temperaturen in einem Bereich zwischen etwa Raumtemperatur und etwa 120° C, wobei die Rückflusstemperatur bevorzugt wird. Die Reaktionszeit beträgt, je nach den übrigen Reaktionsparametern, etwa 1 bis etwa 70 Stunden. Die Ausgangsstoffe der Formel II sind bekannt oder nach an sich bekannten und jedem Fachmann geläufigen Methoden leicht herstellbar.

Gemäss Aspekt b) des erfindungsgemässen Verfahrens wird eine Hydroxygruppe veräthert oder acyliert. Es handelt sich also hierbei um eine Verätherung oder Acylierung einer Hydroxygruppe, und Methoden zur Durchführung einer derartigen Verätherung sind an sich bekannt und jedem Fachmann geläufig. Als Verätherungs- oder Acylierungsmittel verwendet man zweckmässigerweise ein entsprechendes Halogenid, insbesondere ein Chlorid oder Bromid. Im Falle der Verätherung arbeitet man zweckmässigerweise in Gegenwart einer Base, beispielsweise eines Alkalimetallhydroxids, wie Kaliumhydroxid, und in Gegenwart eines unter den Reaktionsbedingungen inerten organischen Lösungsmittels, beispielsweise in N,N-Dimethylformamid, Dimethylsulfoxid, Toluol oder dergleichen. Im Falle der Acylierung arbeitet man zweckmässigerweise in Gegenwart eines säurebindenden Mittels, beispielsweise eines tertiären Amins, wie Pyridin, das gleichzeitig als Lösungsmittel dienen kann. Die Reaktionstemperatur liegt zweckmässigerweise in einem Bereich von etwa -10° C bis etwa 50° C.

Die als Ausgangsstoffe verwendeten Verbindungen der Formel IV können in Analogie zur Verfahrensvariante a) aus Verbindungen der Formel II und Verbindungen der Formel HC≡C-E-OH, worin E obige Bedeutung besitzt, hergestellt werden.

Wie eingangs erwähnt sind die Verbindungen der Formel I neu. Sie besitzen wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben entweder ausgeprägte anxiolytische, antikonvulsive, muskelrelaxierende und sedativ-hypnotische Eigenschaften, und/oder sie antagonisieren selektiv einige oder alle Wirkungen, welche tranquilisierend wirksame 1,4-Benzodiazepine oder andere Substanzen über die zentralen Benzodiazepin-Rezeptoren entfalten, partiell oder vollständig.

Die Affinität von Verbindungen der allgemeinen Formel I zu den zentralen Benzodiazepin-Rezeptoren wurde nach der in Life Science 20, 2101-2110 (1977) und Science 198, 849-851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ ("50% inhibiting concentration") diejenige Konzentration der jeweiligen Testsubstanz, welche eine 50-proz. Hemmung der spezifischen Bindung des tritiierten Diazepams an die spezifischen Benzodiazepin-Rezeptoren im cerebralen Cortex bewirkt.

In der nachfolgenden Tabelle werden die mit repräsentativen Vertretern der durch die allgemeine Formel I definierten Verbindungsklasse erhaltenen Resultate dargestellt.

| Verbindung | Affinität zu Benzodiazepin-Rezeptoren IC 50, nMol/1 |
|---|---|
| A | 1,4 |
| B | 2,6 |
| C | 5,0 |
| D | 1,4 |
| E | 3,7 |
| F | 2,2 |

A = 7-Chlor-3-[3-(Cyclopropylmethoxy)-1-propinyl]-4,5-dihydro-5-methyl-6H-imidazo[1,5-a][1,4]benzodiazepin-6-on

B = 3-(7-Chlor-5,6-dihydro-5-methyl-6-oxo-4H-imidazo-[1,5-a][1,4]benzodiazepin-3-yl)-2-propinyl acetat

C = 7-Chlor-3-[3-(cyclopropylmethoxy)-3-methyl-1-butinyl]-4,5-dihydro-5-methyl-6H-imidazo[1,5-a][1,4]benzodiazepin-6-on

D = 7-Chlor-4,5-dihydro-3-[(Z)-5-hydroxy-3-methyl-3-penten-1-inyl]-5-methyl-6H-imidazo[1,5-a][1,4]benzodiazepin-6-on

E = 7-Chlor-4,5-dihydro-3(3-hydroxy-3-methyl-4-penten-1-inyl]-5-methyl-6H-imidazo[1,5-a][1,4]benzodiazepin-6-on

F = 7-Chlor-4,5-dihydro-3-[(E)-5-hydroxy-3-methyl-3-penten-1-inyl]-5-methyl-6H-imidazo[1,5-a][1,4]benzodiazepin-6-on

Die Verbindungen der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die Verbindungen der Formel I mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I und ein therapeutisch inertes Excipiens, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I und gegebenenfalls einen oder mehrere andere therapeutisch wertvolle Stoffe zusammen mit einem oder mehreren therapeutisch inerten Trägern in eine galenische Darreichungsform bringt.

Wie eingangs erwähnt, können die Verbindungen der Formel I erfindungsgemäss bei der Bekämpfung bzw. Verhütung von Krankheiten verwendet werden und zwar insbesondere bei der Bekämpfung von Konvulsionen und Angstzuständen und/oder bei der partiellen oder vollständigen Antagonisierung einiger oder aller Wirkungen, welche tranquilisierend wirksame 1,4-Benzodiazepine oder andere Substanzen über die zentralen Benzodiazepin-Rezeptoren entfalten. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 0,1 mg bis 100 mg angemessen sein.

Ebenfalls Gegenstand der Erfindung ist schliesslich, wie eingangs erwähnt, die Verwendung von Verbindungen der Formel I für die Herstellung von Arzneimitteln, insbesondere von Arzneimitteln zur Anwendung bei der Bekämpfung bzw. Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

Die nachfolgenden Beispiele sollen die vorliegende Erfindung näher erläutern, ihren Umfang jedoch in keiner Weise beschränken. Sämtliche Temperaturen sind in Celsiusgraden angegeben.

## Beispiel 1

a) 5,20 g (14 mMol) 7-Chlor-4,5-dihydro-3-jod-5-methyl-6H-imidazo[1,5-a][1,4]benzodiazepin-6-on werden mit 1,04 g (18,5 mMol) Propargylalkohol, 70 mg Bis-(triphenylphosphin)-palladium(II)-dichlorid und 10 mg Kupfer(I)-jodid in 35 ml Diäthylamin während 4 Stunden unter Rückfluss zum Sieden erhitzt. Das Reaktionsgemisch wird eingedampft, und der Rückstand wird in Methylenchlorid aufgeschlämmt. Man nutscht die Suspension ab und wäscht den Nutschkuchen mit Essigester. Nach zwei aufeinanderfolgenden Umkristallisationen aus Aethanol bzw. N,N-Dimethylformamid erhält man 7-Chlor-4,5-dihydro-3-(3-hydroxy-1-propinyl)-5-methyl-6H-imidazo[1,5-a][1,4]benzodiazepin-6-on vom Schmelzpunkt 250-252°.

b) 4,5 g (15 mMol) 7-Chlor-4,5-dihydro-3-(3-hydroxy-1-propinyl)-5-methyl -6H-imidazo[1,5-a][1,4]-benzodiazepin-6-on werden in 30 ml Pyridin suspendiert und bei 0 bis 5° C innert 15 Minuten tropfenweise mit 4,1 ml (20 mMol) Caprinsäurechlorid versetzt. Man entfernt das Kühlbad und lässt während 1,5 Stunden bei Raumtemperatur rühren. Das Reaktionsgemisch wird dann auf 300 ml Wasser gegossen und mit 30 ml konzentrierter Salzsäure auf pH 1 angesäuert. Man extrahiert dreimal mit Methylenchlorid, trocknet die vereinigten organischen Auszüge über Magnesiumsulfat und dampft sie ein. Durch Chromatographieren des Rückstandes an Kieselgel unter Eluieren mit Essigester/Methylenchlorid (1/3) und Kristallisieren aus Methylenchlorid, Petroläther und Hexan erhält man 1,55 g (22%) 3-(7-Chlor-5,6-dihydro-5-methyl-6-oxo -4H-imidazo[1,5-a][1,4]benzodiazepin-3-yl)-2-propinyl decanoat vom Schmelzpunkt 70-72°.

In analoger Weise wurden aus 7-Chlor-4,5-dihydro-3-(3-hydroxy-1-propinyl)-5-methyl -6H-imidazo[1,5-a][1,4]benzodiazepin-6-on hergestellt:

c) Mit Pivalinsäurechlorid das 3-(7-Chlor-5,6-dihydro-5-methyl-6-oxo -4H-imidazo[1,5-a][1,4]-benzodiazepin-3-yl)-2-propinyl pivalat vom Schmelzpunkt 143-145° (aus Essigester).

d) Mit Acetylchlorid das 3-(7-Chlor-5,6-dihydro-5-methyl-6-oxo -4H-imidazo[1,5-a][1,4]benzodiazepin-3-yl)-2-propinyl acetat vom Schmelzpunkt 194-195° (aus Methylenchlorid und Essigester).

e) Mit Cyclohexancarbonsäurechlorid das 3-(7-Chlor-5,6-dihydro-5-methyl-6-oxo -4H-imidazo[1,5-a]-[1,4]benzodiazepin-3-yl)-2-propinyl cyclohexancarboxylat vom Schmelzpunkt 143-144° (aus Essigester und Hexan).

f) Mit Benzoylchlorid das 3-(7-Chlor-5,6-dihydro-5-methyl-6-oxo -4H-imidazo[1,5-a][1,4]-benzodiazepin-3-yl)-2-propinyl benzoat vom Schmelzpunkt 152-154° (aus Essigester).

g) Mit Cyclopropancarbonsäurechlorid das 3-(7-Chlor-5,6--dihydro-5-methyl-6-oxo -4H-imidazo[1,5-a]-[1,4]benzodiazepin-3-yl)-2-propinyl cyclopropancarboxylat vom Schmelzpunkt 120-121° (aus Essigester).

## Beispiel 2

a) 3,34 g (59 mMol) frisch pulverisiertes Kaliumhydroxid werden in 25 ml N,N-Dimethylformamid suspendiert und mit 4,0 g (13,2 mMol) 7-Chlor-4,5-dihydro-3-(3-hydroxy-1-propinyl)-5-methyl -6H-imidazo-[1,5-a][1,4]benzodiazepin-6-on versetzt. Nach 5-minütigem Rühren bei Raumtemperatur wird auf 2° gekühlt, worauf man 4,95 g (31,1 mMol) Brommethylcyclopropan dazugibt. Man rührt während 1 Stunde bei Raumtemperatur und giesst dann das Reaktionsgemisch auf Wasser. Man extrahiert dreimal mit Methylenchlorid, wäscht die vereinigten organischen Auszüge mit Wasser, trocknet sie über Magnesiumsulfat und dampft sie ein. Durch Kristallisieren des Rückstandes aus Essigester und Aether erhält man 4,0 g (84%) 7-Chlor-3-[3-(cyclopropylmethoxy)-1-propinyl]-4,5-dihydro -5-methyl-6H-imidazo[1,5-a][1,4]benzodiazepin-6-on vom Schmelzpunkt 124-126°.

In analoger Weise wurden aus 7-Chlor-4,5-dihydro-3-(3-hydroxy-1-propinyl)-5-methyl -6H-imidazo[1,5-a][1,4]benzodiazepin-6-on hergestellt:

b) Mit 3-Methoxybenzylchlorid das 7-Chlor-4,5-dihydro-3-[3-(3-methoxybenzyloxy)-1-propinyl] -5-methyl-6H-imidazo[1,5-a][1,4]benzodiazepin-6-on vom Schmelzpunkt 104-106° (aus Methylenchlorid und Hexan).

c) Mit 4-Methoxybenzylchlorid das 7-Chlor-4,5-dihydro-3-[3-(4-methoxybenzyloxy)-1-propinyl] -5-methyl-6H-imidazo[1,5-a][1,4]benzodiazepin-6-on vom Schmelzpunkt 108-110° (aus Essigester und Hexan).

d) Mit Benzylbromid das 3-[3-(Benzyloxy)-1-propinyl]-7-chlor-4,5-dihydro-5-methyl -6H-imidazo[1,5-a][1,4]benzodiazepin-6-on vom Schmelzpunkt 108-109° (aus Methanol und Aether).

e) Mit Brommethylcyclohexan das 7-Chlor-3-[3-(cyclohexylmethoxy)-1-propinyl]-4,5-dihydro -5-methyl-6H-imidazo[1,5-a][1,4]benzodiazepin-6-on vom Schmelzpunkt 68-71° (aus Essigester/Hexan).

f) Mit Propargylbromid das 7-Chlor-4,5-dihydro-5-methyl-3-[3-(3-propinyloxy) -1-propinyl]-6H-imidazo-[1,5-a][1,4]benzodiazepin-6-on vom Schmelzpunkt 133-134° (aus Methanol und Aether).

g) Mit 2-Chloräthylmethyläther das 7-Chlor-4,5-dihydro-3-[3-(2-methoxyäthoxy)-1-propinyl]-5-methyl-6H-imidazo[1,5-a][1,4]benzodiazepin-6-on vom Schmelzpunkt 105-106° (aus Methanol und Aether).

## Beispiel 3

a) 3,73 g (10 mMol) 7-Chlor-4,5-dihydro-3-jod-5-methyl-6H-imidazo[1,5-a][1,4]benzodiazepin-6-on werden mit 1,10 g (12 mMol) 2-Methyl-3-butin-2-ol und 20 ml Diäthylamin vermischt. Man gibt dann 70 mg Bis-(triphenylphosphin)-palladium(II)-dichlorid und 10 mg Kupfer(I)-jodid zu und rührt während 60 Stunden bei Raumtemperatur. Man dampft das Reaktionsgemisch ein und löst den Rückstand in Methylenchlorid. Die Lösung wird zweimal mit Wasser gewaschen, über Magnesiumsulfat getrocknet und eingedampft. Nach Umkristallisieren des Rückstandes aus Essigester erhält man 7-Chlor-4,5-dihydro-3-(3-hydroxy-3-methyl-1-butinyl)-5 -methyl-6H-imidazo[1,5-a][1,4]benzodiazepin-6-on vom Schmelzpunkt 193-194°.

In Analogie zu Beispiel 2 wurden aus 7-Chlor-4,5-dihydro-3-(3-hydroxy-3-methyl-1-butinyl)-5-methyl -6H-imidazo[1,5-a][1,4]benzodiazepin-6-on hergestellt:

b) Mit 2-Chlorbenzylchlorid das 7-Chlor-3-[3-(2-chlorbenzyloxy)-3-methyl-1-butinyl] -4,5-dihydro-5-methyl-6H-imidazo[1,5-a][1,4]benzodiazepin-6-on vom Schmelzpunkt 142-143° (aus Essigester und Hexan).

c) Mit 4-Chlorbenzylchlorid das 7-Chlor-3-[3-(4-chlorbenzyloxy)-3-methyl-1-butinyl] -4,5-dihydro-5-methyl-6H-imidazo[1,5-a][1,4]benzodiazepin-6-on vom Schmelzpunkt 107-108° (aus Essigester und Hexan).

d) Mit 3-Chlorbenzylbromid das 7-Chlor-3-[3-(3-chlorbenzyloxy)-3-methyl-1-butinyl] -4,5-dihydro-5-methyl-6H-imidazo[1,5-a][1,4]benzodiazepin-6-on vom Schmelzpunkt 161-163° (aus Essigester und Hexan).

e) Mit Benzylbromid das 3-[3-(Benzyloxy)-3-methyl-1-butinyl]-7-chlor-4,5-dihydro -5-methyl-6H-imidazo[1,5-a] [1,4]benzodiazepin-6-on vom Schmelzpunkt 143-144° (aus Essigester und Aether).

f) Mit Brommethylcyclopropan das 7-Chlor-3-[3-(cyclopropylmethoxy)-3-methyl-1-butinyl] -4,5-dihydro-5-methyl-6H-imidazo[1,5-a][1,4]benzodiazepin-6-on vom Schmelzpunkt 138-140° (aus Essigester).

g) In Analogie zu Beispiel 1b) wurde aus 7-Chlor-4, 5-dihydro-3-(3-hydroxy-3-methyl-1-butinyl) -5-methyl-6H-imidazo[1,5-a][1,4]benzodiazepin-6-on das 3-(7-Chlor-5,6-dihydro-5-methyl-6-oxo -4H-imidazo-[1,5-aJ[1,4]benzodiazepin-3-yl)-1,1-dimethyl -2-propinyl cyclopropancarboxylat vom Schmelzpunkt 139-141° (aus Essigester und Hexan) hergestellt.

## Beispiel 4

a) 3,30 g (10 mMol) 7-Chlor-4,5-dihydro-3-(3-hydroxy-3-methyl-1-butinyl)-5-methyl -6H-imidazo[1,5-a][1,4]benzodiazepin-6-on werden in 20 ml Pyridin gelöst und bei Raumtemperatur mit 1,57 g (13 mMol) Pivalinsäurechlorid versetzt. Man rührt während 24 Stunden bei 65° und dampft dann das Reaktionsgemisch ein. Man nimmt den Rückstand in Methylenchlorid auf, wäscht die Lösung einmal mit 1N Salzsäure und zweimal mit Wasser, trocknet sie über Magnesiumsulfat und dampft sie ein. Durch Chromatographieren an Kieselgel unter Eluieren mit Essigester und anschliessendes Kristallisieren aus Essigester und Hexan erhält man 0,96 g (23%) 3-(7-Chlor-5,6-dihydro-5-methyl-6-oxo -4H-imidazo[1,5-a][1,4]benzodiazepin-3-yl) -1,1-dimethyl-2-propinyl pivalat vom Schmelzpunkt 132-133°.

In analoger Weise wurden aus 7-Chlor-4,5-dihydro-3-(3-hydroxy-1-butinyl)-5-methyl-6H-imidazo[1,5-a]-[1,4]benzodiazepin-6-on hergestellt:

b) Mit Acetylchlorid zwischen 4° und Raumtemperatur während 2,5 Stunden das 3-(7-Chlor-5,6-dihydro-5-methyl-6-oxo-4H-imidazo[1,5-a][1,4]benzodiazepin-3-yl)-1-methyl-2-propinylacetat vom Schmelzpunkt 144-145° (aus Essigester und Hexan). Ausbeute: 2,86 g (80%).

c) Mit Cyclopropancarbonsäurechlorid zwischen 4° und Raumtemperatur über Nacht das 3-(7-Chlor-5,6-dihydro-5-methyl-6-oxo-4H-imidazo[1,5-a][1,4]benzodiazepin-3-yl)-1-methyl-2-propinylcyclopropancarboxylat vom Schmelzpunkt 158-160° (aus Essigester und Hexan). Ausbeute: 2,75 g (71%).

d) Mit Pivalinsäurechlorid bei 55-60° während 4 Stunden das 3-(7-Chlor-5,6-dihydro-5-methyl-6-oxo-4H-imidazo[1,5-a][1,4]benzodiazepin-3-yl)-1-methyl-2-propinylpivalat vom Schmelzpunkt 138-140° (aus Essigester und Hexan). Ausbeute: 2,75 g (84%).

## Beispiel 5

a) 7,47 g (20 mMol) 7-Chlor-4,5-dihydro-3-jod-5-methyl-6H-imidazo[1,5-a][1,4]benzodiazepin-6-on werden mit 1,75 g (25 mMol) 3-Butin-2-ol, 70 mg Bis-(triphenylphosphin)-palladium(II)-dichlorid und 10 mg Kupfer(I)-jodid in 50 ml Diäthylamin und 20 ml Aethylenchlorid während 4 Stunden unter Rückfluss zum Sieden erhitzt. Nach Abdampfen des Lösungsmittels nimmt man den Rückstand in Methylenchlorid auf und nutscht die so erhaltene Suspension ab. Man wäscht das erhaltene Material mit Methylenchlorid und erhält nach Umkristallisieren aus Essigester 7-Chlor-4,5-dihydro-3-(3-hydroxy-1-butinyl) -5-methyl-6H-imidazo[1,5-a][1,4]benzodiazepin-6-on vom Schmelzpunkt 251-252° C.

In Analogie zu Beispiel 2 wurden aus 7-Chlor-4,5-dihydro-3-(3-hydroxy-1-butinyl)-5-methyl-6H-imidazo-[1,5-a][1,4]benzodiazepin-6-on hergestellt:

b) Mit Benzylbromid das 3-[3-(Benzyloxy)-1-butinyl]-7-chlor-4,5-dihydro -5-methyl-6H-imidazo[1,5-a]-[1,4]benzodiazepin-6-on vom Schmelzpunkt 150-151° (aus Methylenchlorid und Hexan). Ausbeute: 0,82 g (63%).

c) Mit Brommethylcyclopropan bei 5° bei Raumtemperatur während 2,5 Stunden das 7-Chlor-3-[3-(cyclopropylmethoxy)-1-butinyl]-4,5-dihydro-5-methyl-6H-imidazo[1,5-a][1,4]benzodiazepin-6-on vom Schmelzpunkt 138-139° (aus Methanol und Aether). Ausbeute: 2,17 g (58%).

## Beispiel 6

a) 25 g (85 mMol) Aethyl 5,6-dihydro-5,7-dimethyl-6-oxo-4H-imidazo[1,5-a][1,4]benzodiazepin-3-carboxylat werden mit 3,40 g (85 mMol) Natriumhydroxid in 200 ml Aethanol und 15 ml Wasser während 1 Stunde unter Rückfluss zum Sieden erhitzt. Nach Eindampfen des Aethanols wird mit Wasser verdünnt und mit 21 ml 4-normaler Salzsäure angesäuert. Man nutscht die erhaltene Suspension ab und wäscht sie mit Wasser. Nach Trocknen des Nutschkuchens erhält man 5,6-Dihydro-5,7-dimethyl-6-oxo-4H-imidazo[1,5-a]-[1,4]benzodiazepin-3-carbonsäure vom Zersetzungspunkt 274-275° C.

b) 22,20 g (81,8 mMol) 5,6-Dihydro-5,7-dimethyl-6-oxo-4H-imidazo[1,5-a][1,4]benzodiazepin-3-carbonsäure werden bis zur Beendigung der $CO_2$-Abspaltung im Metallbad auf 290-300° C erhitzt. Die Schmelze wird in Methylenchlorid und Aethanol gelöst und die Lösung konzentriert bis kein Methylenchlorid mehr überdestilliert. Aus dieser Lösung kristallisiert das 4,5-Dihydro-5,7-dimethyl-6H-imidazo[1,5-a][1,4]-benzodiazepin-6-on vom Schmelzpunkt 224-225° C.

c) 15,85 g (69,7 mMol) 4,5-Dihydro-5,7-dimethyl-6H-imidazo[1,5-a][1,4]benzodiazepin-6-on werden mit 67 g (264 mMol) Jod in 100 ml N,N-Dimethylformamid während 2,5 Stunden auf 95° C erhitzt. Das

Reaktionsgemisch wird dann auf 450 ml Wasser gegossen, mit Methylenchlorid versetzt, mit Natrium thiosulfat entfärbt und mit Natriumbicarbonat neutralisiert. Die wässrige Phase wird abgetrennt und sechsmal mit Methylenchlorid extrahiert. Man wäscht die vereinigten organischen Phasen dreimal mit Wasser, trocknet sie über Magnesiumsulfat und dampft sie ein. Durch Chromatographieren des Rückstandes an Kieselgel unter Eluieren mit Essigester und Umkristalisieren aus Essigester und Hexan erhält man 4,5-Dihydro-3-jod-5,7-dimethyl-6H-imidazo[1,5-a][1,4]benzodiazepin-6-on vom Schmelzpunkt 106-108 °C.

d) 5 g (14,2 mMol) 4,5-Dihydro-3-jod-5,7-dimethyl-6H-imidazo[1,5-a][1,4]benzodiazepin-6-on werden mit 1,50 g (17,8 mMol) 2-Methyl-3-butin-2-ol, 70 mg Bis-(triphenylphosphin)-palladium(II)-dichlorid und 20 mg Kupfer(I)-jodid in 40 ml Diäthylamin während 4,5 Stunden unter Rückfluss zum Sieden erhitzt. Das Reaktionsgemisch wird dann eingedampft, und der Rückstand wird an Kieselgel chromatographiert unter Eluieren mit Essigester. Durch Umkristallisieren aus Essigester erhält man 4,5-Dihydro-3-(3-hydroxy-3-methyl-1-butinyl)-5,7-dimethyl-6H-imidazo[1,5-a][1,4]benzodiazepin-6-on vom Schmelzpunkt 165-167 °C.

e) In Analogie zu Beispiel 2 wurde aus 4,5-Dihydro-3-(3-hydroxy-3-methyl-1-butinyl)-5,7-dimethyl -6H-imidazo[1,5-a][1,4]benzodiazepin-6-on und Benzylchlorid das 3-(3-Benzyloxy-3-methyl-1-butinyl)-4, 5-dihydro- 5,7-dimethyl-6H-imidazo[1,5-a][1,4]benzodiazepin-6-on vom Schmelzpunkt 134° (aus Essigester und Hexan) hergestellt.

## Beispiel 7

a) 27,3 g (100 mMol) (S)-8-Chlor-11,12,13,13a-tetrahydro-9H-imidazo[1,5-a]pyrrolo[2,1-c][1,4]-benzodiazepin-9-on werden mit 88 g (350 mMol) Jod in 200 ml N,N-Dimethylformamid während 3 Stunden bei 100° gerührt. Man kühlt das Reaktionsgemisch ab, filtriert das ausgefallene Produkt ab, wäscht es mit Essigester nach und erhält nach Trocknen (S)-8-Chlor- 11,12,13,13a-tetrahydro-1-jod-9H -imidazo[1,5-a]-pyrrolo[2,1-c][1,4]benzodiazepin-9-on vom Schmelzpunkt 298-300°.

b) 3,99 g (10 mMol) (S)-8-Chlor-11,12,13,13a-tetrahydro-1-jod-9H-imidazo[1,5-a]pyrrolo[2,1-c][1,4]-benzodiazepin-9-on werden über Nacht mit 0,88 g (10,5 mMol) 2-Methyl-3-butin-2-ol, 25 mg Palladium(II)-acetat, 100 mg Triphenylphosphin und 10 mg Kupfer(I)-jodid in 40 ml Triäthylamin und 20 ml N,N-Dimethylformamid bei 100° gerührt. Man dampft dann das Gemisch zur Trockene ein und chromatographiert den Rückstand an Kieselgel unter Eluieren mit Essigester. Nach Umkristallisieren aus Essigester erhält man (S)-8-Chlor-11,12,13,13a-tetrahydro-1-(3-hydroxy-3-methyl -1-butinyl)-9H-imidazo[1,5-a]pyrrolo-[2,1-c][1,4]benzodiazepin -9-on vom Schmelzpunkt 234-235°.

c) In Analogie zu Beispiel 2 wurde aus (S)-8-Chlor-11,12,13,13a-tetrahydro-1-(3-hydroxy-3-methyl -1-butinyl)-9H-imidazo[1,5-a]pyrrolo[2,1-c][1,4]benzodiazepin -9-on und Benzylbromid das (S)-1-(3-Benzyloxy-3-methyl-1-butinyl)-8-chlor-11,12,13,13a -tetrahydro-9H-imidazo[1,5-a]pyrrolo[2,1-c][1,4]benzodiazepin -9-on vom Schmelzpunkt 146-148° (aus Essigester und Hexan) hergestellt.

## Beispiel 8

a) 7,47 g (20 mMol) 7-Chlor-4,5-dihydro-3-jod-5-methyl-6H-imidazo[1,5-a][1,4]benzodiazepin-6-on, 2,88 g (30 mMol) 3-Methyl-1-penten-4-in-3-ol, 110 mg Bis(triphenylphosphin)palladium(II)dichlorid und 35 mg Kupfer(I)jodid werden in 60 ml Triäthylamin und 30 ml Dimethylformamid während 12 Stunden unter Rückfluss zum Sieden erhitzt. Man dampft das Reaktionsgemisch ein und chromatographiert den Rückstand an Kieselgel unter Eluieren mit Essigester. Durch Umkristallisieren des Rückstandes aus Acetonitril erhält man 4,34 g (63%) 7-Chlor-4,5-dihydro-3-(3-hydroxy-3-methyl-4-penten-1-inyl) -5-methyl-6H-imidazo-[1,5-a][1,4]benzodiazepin- 6-on vom Schmelzpunkt 185-187°.

In analoger Weise wurden aus 7-Chlor-4,5-dihydro-3-jod-5-methyl -6H-imidazo[1,5-a][1,4]benzodiazepin-6-on hergestellt:

b) Mit cis-3-Methyl-2-penten-4-in-1-ol das 7-Chlor-4,5-dihydro-3-[(Z)-5-hydroxy-3-methyl-3-penten-1-inyl]-5-methyl-6H-imidazo[1,5-a][1,4]benzodiazepin-6-on vom Schmelzpunkt 193-194° (aus Methylenchlorid und Essigester).

c) Mit trans-3-Methyl-2-penten-4-in-1-ol das 7-Chlor-4,5-dihydro-3-[(E)-5-hydroxy-3-methyl-3-penten -1-inyl]-5-methyl-6H-imidazo[1,5-a][1,4]benzodiazepin-6-on vom Schmelzpunkt 186-188° (aus Essigester und Acetonitril).

d) Mit Methyl-propargyl-sulfid und Diäthylamin anstelle von Triäthylamin das 7-Chlor-4,5-dihydro-5-methyl-3-[3-(methylthio)-1-propinyl] -6H-imidazo[1,5-a][1,4]benzodiazepin-6-on vom Schmelzpunkt 165-166° (aus Essigester).

e) Mit 3-Butinyl-methyl-sulfid und Diäthylamin anstelle von Triäthylamin das 7-Chlor-4,5-dihydro-5-methyl-3-[4-(methylthio)-1-butinyl] -6H-imidazo[1,5-a][1,4]benzodiazepin-6-on vom Schmelzpunkt 143-145° (aus Essigester).

f) Mit 2-Phenyläthyl-propargyl-äther und Diäthylamin anstelle von Triäthylamin das 7-Chlor-4,5-dihydro-5-methyl-3-[3-(phenäthyloxy)-1-propinyl] -6H-imidazo[1,5-a][1,4]benzodiazepin-6-on vom Schmelzpunkt 76-78° (aus Essigester und Hexan).

## Beispiel 9

a) 2,31 g (10 mMol) 8-Fluor-4,5-dihydro-5-methyl-6H-imidazo[1,5-a][1,4]benzodiazepin-6-on werden mit 8,88 g (35 mMol) Jod in 25 ml N,N-Dimethylformamid während 1,5 Stunden bei 95° gerührt. Man giesst dann das Reaktionsgemisch auf 300 ml Wasser, entfärbt es mit einer Natriumthiosulfat-Lösung und extrahiert es viermal mit Methylenchlorid. Die organischen Auszüge werden dreimal mit Wasser gewaschen, über Magnesiumsulfat getrocknet und eingedampft. Nach Umkristallisieren des Rückstandes aus Essigester erhält man 8-Fluor-4,5-dihydro-3-jod-5-methyl-6H -imidazo[1,5-a][1,4]benzodiazepin-6-on vom Schmelzpunkt 187-188°.

b) In Analogie zu Beispiel 8a) wurde aus 8-Fluor-4,5-dihydro-3-jod-5-methyl -6H-imidazo[1,5-a][1,4]-benzodiazepin-6-on, Benzyl-propargyl-äther und Diäthylamin anstelle von Triäthylamin das 3-[3-(Benzyloxy)-1-propinyl]-4,5-dihydro-8-fluor-5-methyl -6H-imidazo[1,5-a][1,4]benzodiazepin-6-on vom Schmelzpunkt 90-91° (aus Essigester und Hexan) hergestellt.

## Beispiel 10

a) 19,1 g (56,8 mMol) 7-Brom-5,6-dihydro-5-methyl-6-oxo-4H-imidazo[1,5-a][1,4]benzodiazepin-3-carbonsäure werden bei 290-300° decarboxyliert. Man nimmt die Schmelze in Methylenchlorid auf, verdünnt die Lösung mit Essigester und Aethanol und entfärbt sie mit Tierkohle. Nach Eindampfen und Umkristallisieren aus Essigester und Aethanol erhält man 7-Brom-4,5-dihydro-5-methyl -6H-imidazo[1,5-a][1,4]-benzodiazepin-6-on vom Schmelzpunkt 196-197°.

b) 12,80 g (44 mMol) 7-Brom-4,5-dihydro-5-methyl -6H-imidazo[1,5-a][1,4]benzodiazepin-6-on werden mit 39 g (154 mMol) Jod in 80 ml N,N-Dimethylformamid während 3,5 Stunden bei 95° gerührt. Man dampft das Reaktionsgemisch ein, nimmt den Rückstand in Methylenchlorid und Wasser auf und entfärbt durch Zugabe von Natriumthiosulfat. Man filtriert, trennt die organische Phase ab, trocknet sie über Magnesiumsulfat und dampft sie ein. Nach Chromatographieren des Rückstandes an Kieselgel unter Eluieren mit Essigester und Umkristalli sieren aus Methylenchlorid und Essigester erhält man 7-Brom-4,5-dihydro-3-jod-5-methyl -6H-imidazo[1,5-a][1,4]benzodiazepin-6-on vom Schmelzpunkt 203-204°.

c) In Analogie zu Beispiel 8a) wurde aus 7-Brom-4,5-dihydro-3-jod-5-methyl -6H-imidazo[1,5-a][1,4]-benzodiazepin-6-on, Benzyl-propargyl-äther und Diäthylamin anstelle von Triäthylamin das 3-[3-(Benzyloxy)-1-propinyl]-7-brom-4,5-dihydro-5-methyl -6H-imidazo[1,5-a][1,4]benzodiazepin-6-on vom Schmelzpunkt 106-107° (aus Methanol und Aether) hergestellt.

## Beispiel 11

In Analogie zu Beispiel 8a) wurde aus 4,5-Dihydro-3-jod-5-methyl -6H-imidazo[1,5-a][1,4]benzodiazepin-6-on, Benzyl-propargyl-äther und Diäthylamin anstelle von Triäthylamin das 3-[3-(Benzyloxy)-1-propinyl]-4,5-dihydro-5-methyl-6H-imidazo[1,5-a][1,4]benzodiazepin-6-on vom Schmelzpunkt 61-63° (aus Aethanol und Aether) erhalten.

## Beispiel A

Es werden in üblicher Weise Tabletten folgender Zusammensetzung hergestellt:

| | mg/Tablette |
|---|---|
| 7-Chlor-3-[3-(cyclopropylmethoxy)-1-propinyl]-4,5-dihydro-5-methyl-6H-imidazo-[1,5-a][1,4]benzodiazepin-6-on | 0,2 |
| Milchzucker | 140 |
| Maisstärke | 50,8 |
| Polyvinylpyrrolidin | 8 |
| Magnesiumstearat | 1 |
| Tablettengewicht | 200 |

Beispiel B

Es werden in üblicher Weise Kapseln folgender Zusammensetzung hergestellt:

| | mg/Kapsel |
|---|---|
| 7-Chlor-3-[3-(cyclopropylmethoxy)-1-propinyl]-4,5-dihydro-5-methyl-6H-imidazo-[1,5-a][1,4]benzodiazepin-6-on | 0,5 |
| Milchzucker | 40 |
| Maisstärke | 8 |
| Talk | 1 |
| Magnesiumstearat | 0,5 |
| Kapselfüllgewicht | 50 |

Beispiel C

Es werden Injektionslösungen folgender Zusammensetzung hergestellt:

| | |
|---|---|
| 7-Chlor-3-[3-(cyclopropylmethoxy)-1-propinyl]-4,5-dihydro-5-methyl-6H-imidazo-[1,5-a][1,4]benzodiazepin-6-on | 0,1 mg |
| Natriumchlorid | 45,0 mg |
| SESQUESTREN $Na_2$ | 0,5 mg |
| Essigsäure p.a. | 0,5 mg |
| NaOH 1N ad pH 4,5 | q.s. |
| Wasser zu Injektionszwecken q.s. ad | 5,0 ml |

## Ansprüche

1. Verbindungen der allgemeinen Formel

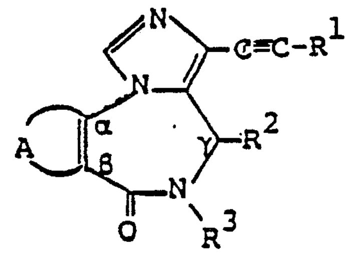

I

worin A zusammen mit den beiden mit α und β bezeichneten Kohlenstoffatomen eine der Gruppen

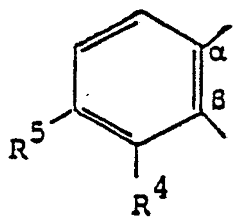
(a)
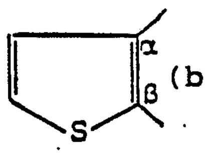
(b) und
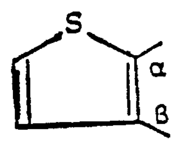
(c),

$R^1$ eine gegebenenfalls durch Hydroxy, Oxo, Aryl oder die Gruppe -OR, -SR oder -OCOR substituierte, partiell ungesättigte niedere Kohlenwasserstoffgruppe oder eine durch Aryl oder die Gruppe -OR′, -SR′ oder -OCOR′ substituierte niedere Alkylgruppe, R und R′ je Aryl oder eine gegebenenfalls durch Aryl oder niederes Alkoxy substituierte, gesättigte oder partiell ungesättigte $C_{1-18}$-Kohlenwasserstoff- gruppe, $R^2$ Wasserstoff und $R^3$ niederes Alkyl oder $R^2$ und $R^3$ zusammen Dimethylen oder Trimethylen und $R^4$ und $R^5$ je Wasserstoff, Halogen, Trifluormethyl, Cyano, Nitro oder niederes Alkyl bedeuten, wobei die Gruppe -OR′ eine von niederem Alkoxy verschiedene Bedeutung hat und wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen oder Trimethylen bedeuten.

2. Verbindungen nach Anspruch 1, worin R und R′ je Aryl oder eine gegebenenfalls durch Aryl substituierte, gesättigte oder partiell ungesättigte $C_{1-18}$-Kohlenwasserstoffgruppe bedeuten.

3. Verbindungen nach Anspruch 1 oder 2, worin $R^1$ eine gegebenenfalls durch Hydroxy, Oxo, Aryl oder die Gruppe -OR, -SR oder -OCOR substituierte niedere Alkenylgruppe oder eine durch Aryl oder die Gruppe -OR′, -SR′ oder -OCOR′ substituierte niedere Alkylgruppe bedeutet.

4. Verbindungen nach Anspruch 3, worin $R^1$ eine durch Hydroxy oder die Gruppe -OR, -SR oder -OCOR substituierte niedere Alkenylgruppe oder eine durch die Gruppe -OR′, -SR′ oder -OCOR′ substituierte niedere Alkylgruppe bedeutet.

5. Verbindungen nach einem der Ansprüche 1-4, worin R und R′ je Aryl, Aryl-niederes Alkyl, niederes Alkoxy-niederes Alkyl, niederes Alkyl, $C_{3-7}$-Cycloalkyl oder $C_{3-7}$-Cycloalkyl-niederes Alkyl bedeuten.

6. Verbindungen nach einem der Ansprüche 1-5, worin $R^2$ Wasserstoff und $R^3$ Methyl bedeuten oder worin $R^2$ und $R^3$ zusammen Dimethylen oder Trimethylen bedeuten und das mit γ bezeichnete Kohlenstoffatom die (S)-Konfiguration aufweist.

7. Verbindungen nach einem der Ansprüche 1-6, worin A einen Rest der Formel (a) und eines von $R^4$ und $R^5$ Wasserstoff und das andere Wasserstoff oder Halogen bedeuten.

8. Verbindungen nach Anspruch 7, worin $R^4$ und $R^5$ beide Wasserstoff oder $R^4$ Wasserstoff und $R^5$ Fluor oder $R^4$ Chlor oder Brom und $R^5$ Wasserstoff bedeuten.

9. 7-Chlor-3-[3-(cyclopropylmethoxy)-1-propinyl]-4,5-dihydro-5-methyl-6H-imidazo[1,5-a][1,4]-benzodiazepin-6-on.

10. 3-(7-Chlor-5,6-dihydro-5-methyl-6-oxo-4H-imidazo[1,5-a][1,4]benzodiazepin-3-yl)-2-propinyl acetat.

11. 7-Chlor-3-[3-(cyclopropylmethoxy)-3-methyl-1-butinyl]-4,5-dihydro-5-methyl-6H-imidazo[1,5-a][1,4]-

benzodiazepin-6-on.

12. 7-Chlor-4,5-dihydro-3-[(Z)-5-hydroxy-3-methyl-3-penten-1-inyl]-5-methyl-6H-imidazo[1,5-a][1,4]-benzodiazepin-6-on.

13. 7-Chlor-4,5-dihydro-3-(3-hydroxy-3-methyl-4-penten-1-inyl)-5-methyl-6H-imidazo[1,5-a] [1,4]-benzodiazepin-6-on.

14. 7-Chlor-4,5-dihydro-3-[(E)-5-hydroxy-3-methyl-3-penten-1-inyl]-5-methyl-6H-imidazo[1,5-a][1,4]-benzodiazepin-6-on.

15. Verbindungen gemäss einem der Ansprüche 1 bis 14 zur Anwendung als therapeutische Wirkstoffe.

16. Verbindungen gemäss einem der Ansprüche 1 bis 14 zur Anwendung als therapeutische Wirkstoffe, welche antikonvulsiv, anxiolytisch, muskelrelaxierend und sedativ-hypnotisch wirksam sind und/oder selektiv einige oder alle Wirkungen, welche tranquilisierend wirksame 1,4-Benzodiazepine oder andere Substanzen über die zentralen Benzodiazepin-Rezeptoren entfalten, partiell oder vollständig antagonisieren.

17. Verfahren zur Herstellung von Verbindungen gemäss einem der Ansprüche 1 bis 14, dadurch gekennzeichnet, dass man

a) eine Verbindung der allgemeinen Formel

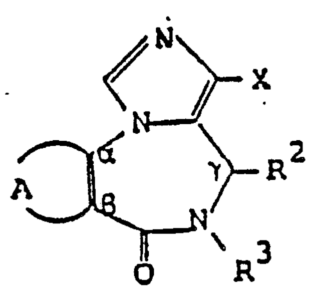

II

worin A, $R^2$ und $R^3$ die in Anspruch 1 angegebene Bedeutung besitzen, und X Brom oder Jod bedeutet, wobei aber im Falle, dass A einen Rest der Formel (a) und $R^4$ und/oder $R^5$ Halogen bedeuten, dieses Halogen Fluor oder Chlor ist, wenn X Brom bedeutet, bzw. Fluor, Chlor oder Brom ist, wenn X Jod bedeutet,

mit einer Verbindung der allgmeinen Formel

$HC{\equiv}C\text{-}R^1$ III

worin $R^1$ die in Anspruch 1 angegebene Bedeutung besitzt,

umsetzt; oder

b) eine Verbindung der Formel I, worin $R^1$ eine durch Hydroxy substituierte, partiell ungesättigte niedere Kohlenwasserstoffgruppe bedeutet, oder eine Verbindung der allgemeinen Formel

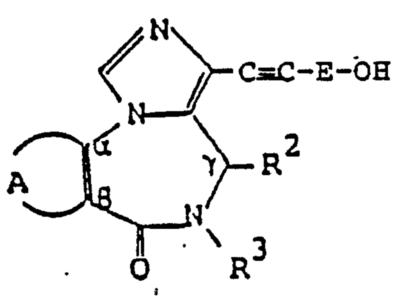

IV

worin E niederes Alkylen bedeutet, und $R^2$, $R^3$ und A die in Anspruch 1 angegebene Bedeutung besitzen, mit einem die Gruppe -OR oder -OCOR, bzw. -OR′ oder -OCOR′ liefernden Mittel veräthert bzw. acyliert.

18. Arzneimittel, enthaltend eine Verbindung gemäss einem der Ansprüche 1 bis 14 und einen therapeutisch inerten Träger.

19. Arzneimittel gemäss Anspruch 18, welche antikonvulsiv, anxiolytisch, muskelrelaxierend und sedativ-hypnotisch wirksam sind und/oder selektiv einige oder alle Wirkungen, welche tranquilisierend wirksame 1,4-Benzodiazepine oder andere Substanzen über die zentralen Benzodiazepin-Rezeptoren entfalten, partiell oder vollständig antagonisieren.

20. Verwendung von Verbindungen gemäss einem der Ansprüche 1 bis 14 bei der Bekämpfung oder Verhütung von Krankheiten.

21. Verwendung von Verbindungen gemäss einem der Ansprüche 1 bis 14 bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

22. Verwendung von Verbindungen gemäss einem der Ansprüche 1 bis 14 für die Herstellung von Arzneimitteln zur Anwendung bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.

Patentansprüche für folgende Vertragsstaaten: GR, ES

1. Verfahren zur Herstellung von Verbindungen der allgemeinen Formel

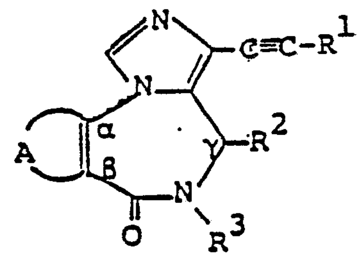

I

worin A zusammen mit den beiden mit α und β bezeichneten Kohlenstoffatomen eine der Gruppen

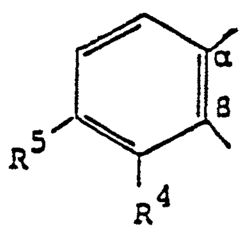 (a) 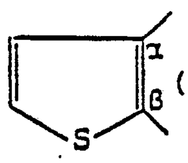 (b) und 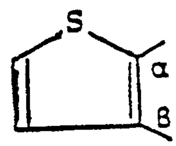 (c),

$R^1$ eine gegebenenfalls durch Hydroxy, Oxo, Aryl oder die Gruppe -OR, -SR oder -OCOR substituierte, partiell ungesättigte niedere Kohlenwasserstoffgruppe oder eine durch Aryl oder die Gruppe -OR′, -SR′ oder -OCOR′ substituierte niedere Alkylgruppe, R und R′ je Aryl oder eine gegebenenfalls durch Aryl oder niederes Alkoxy substituierte, gesättigte oder partiell ungesättigte $C_{1-18}$-Kohlenwasserstoffgruppe, $R^2$ Wasserstoff und $R^3$ niederes Alkyl oder $R^2$ und $R^3$ zusammen Dimethylen oder Trimethylen und $R^4$ und $R^5$ je Wasserstoff, Halogen, Trifluormethyl, Cyano, Nitro oder niederes Alkyl bedeuten, wobei die Gruppe -OR′ eine von niederem Alkoxy verschiedene Bedeutung hat und wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen oder Trimethylen bedeuten,
dadurch gekennzeichnet, dass man
a) eine Verbindung der allgemeinen Formel

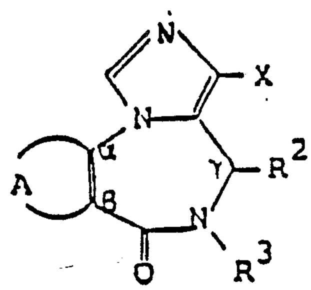

II

worin A, $R^2$ und $R^3$ obige Bedeutung besitzen, und X Brom oder Jod bedeutet, wobei aber im Falle, dass A einen Rest der Formel (a) und $R^4$ und/oder $R^5$ Halogen bedeuten, dieses Halogen Fluor oder Chlor ist, wenn X Brom bedeutet, bzw. Fluor, Chlor oder Brom ist, wenn X Jod bedeutet,
mit einer Verbindung der allgmeinen Formel

$HC{\equiv}C\text{-}R^1$ III

worin $R^1$ obige Bedeutung besitzt,
umsetzt; oder
b) eine Verbindung der Formel I, worin $R^1$ eine durch Hydroxy substituierte, partiell ungesättigte niedere Kohlenwasserstoffgruppe bedeutet, oder eine Verbindung der allgemeinen Formel

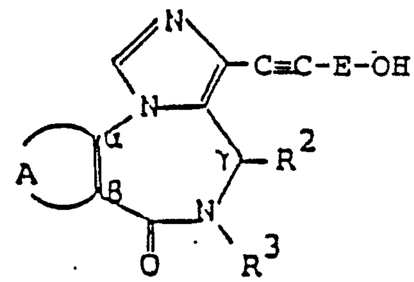

IV

worin E niederes Alkylen bedeutet, und $R^2$, $R^3$ und A obige Bedeutung besitzen,
mit einem die Gruppe -OR oder -OCOR, bzw. -OR′ oder -OCOR′ liefernden Mittel veräthert bzw. acyliert.

2. Verfahren nach Anspruch 1, worin R und R′ je Aryl oder eine gegebenenfalls durch Aryl substituierte, gesättigte oder partiell ungesättigte $C_{1-18}$-Kohlenwasserstoffgruppe bedeuten.

3. Verfahren nach Anspruch 1 oder 2, worin $R^1$ eine gegebenenfalls durch Hydroxy, Oxo, Aryl oder die Gruppe -OR, -SR oder -OCOR substituierte niedere Alkenylgruppe oder eine durch Aryl oder die Gruppe -OR′, -SR′ oder -OCOR′ substituierte niedere Alkylgruppe bedeutet.

4. Verfahren nach Anspruch 3, worin $R^1$ eine durch Hydroxy oder die Gruppe -OR, -SR oder -OCOR substituierte niedere Alkenylgruppe oder eine durch die Gruppe -OR′, -SR′ oder -OCOR′ substituierte niedere Alkylgruppe bedeutet.

5. Verfahren nach einem der Ansprüche 1-4, worin R und R′ je Aryl, Aryl-niederes Alkyl, niederes Alkoxy-niederes Alkyl, niederes Alkyl, $C_{3-7}$-Cycloalkyl oder $C_{3-7}$-Cycloalkyl-niederes Alkyl bedeuten.

6. Verfahren nach einem der Ansprüche 1-5, worin $R^2$ Wasserstoff und $R^3$ Methyl bedeuten oder worin $R^2$ und $R^3$ zusammen Dimethylen oder Trimethylen bedeuten und das mit $\gamma$ bezeichnete Kohlenstoffatom die (S)-Konfiguration aufweist.

7. Verfahren nach einem der Ansprüche 1-6, worin A einen Rest der Formel (a) und eines von $R^4$ und $R^5$ Wasserstoff und das andere Wasserstoff oder Halogen bedeuten.

8. Verfahren nach Anspruch 7, worin $R^4$ und $R^5$ beide Wasserstoff oder $R^4$ Wasserstoff und $R^5$ Fluor oder $R^4$ Chlor oder Brom und $R^5$ Wasserstoff bedeuten.

9. Verfahren nach Anspruch 1, dadurch gekennzeichnet, dass man 7-Chlor-3-[3-(cyclopropylmethoxy)-1-propinyl]-4,5-dihydro-5-methyl-6H-imidazo[1,5-a][1,4]benzodiazepin-6-on herstellt.

10. Verfahren nach Anspruch 1, dadurch gekennzeichnet, dass man 3-(7-Chlor-5,6-dihydro-5-methyl-6-oxo-4H-imidazo[1,5-a][1,4]benzodiazepin-3-yl)-2-propinyl acetat herstellt.

11. Verfahren nach Anspruch 1, dadurch gekennzeichnet, dass man 7-Chlor-3-[3-(cyclopropylmethoxy)-3-methyl-1-butinyl]-4,5-dihydro-5-methyl-6H-imidazo[1,5-a][1,4]benzodiazepin-6-on herstellt.

12. Verfahren nach Anspruch 1, dadurch gekennzeichnet, dass man 7-Chlor-4,5-dihydro-3-[(Z)-5-hydroxy-3-methyl-3-penten-1-inyl]-5-methyl-6H-imidazo[1,5-a][1,4]benzodiazepin-6-on herstellt.

13. Verfahren nach Anspruch 1, dadurch gekennzeichnet, dass man 7-Chlor-4,5-dihydro-3-(3-hydroxy-3-methyl-4-penten-1-inyl)-5-methyl-6H-imidazo[1,5-a][1,4]benzodiazepin-6-on herstellt.

14. Verfahren nach Anspruch 1, dadurch gekennzeichnet, dass man 7-Chlor-4,5-dihydro-3-[(E)-5-hydroxy-3-methyl-3-penten-1-inyl]-5-methyl-6H-imidazo[1,5-a][1,4]benzodiazepin-6-on herstellt.

15. Verfahren zur Herstellung von Arzneimitteln, insbesondere von Mitteln, welche antikonvulsiv, anxiolytisch, muskelrelaxierend und sedativ-hypnotisch wirksam sind und/oder selektiv einige oder alle Wirkungen, welche tranquilisierend wirksame 1,4-Benzodiazepine oder andere Substanzen über die zentralen Benzodiazepin-Rezeptoren entfalten, partiell oder vollständig antagonisieren, dadurch gekennzeichnet, dass man eine oder mehrere Verbindungen der in Anspruch 1 definierten Formel I und erwünschtenfalls eine oder mehrere andere therapeutisch wertvolle Stoffe zusammen mit einem therapeutisch inerten Träger

in eine galenische Darreichungsform bringt.

16. Verwendung von Verbindungen der in Anspruch 1 definierten Formel I zur Herstellung von Arzneimitteln für die Anwendung bei der Bekämpfung oder Verhütung von Konvulsionen, Angstzuständen, Spannungszuständen, Erregungszuständen und Schlafstörungen und/oder bei der partiellen oder vollständigen selektiven Antagonisierung einiger oder aller Wirkungen, welche tranquilisierend wirksame 1,4-Benzodiazepine oder andere Substanzen über die zentralen Benzodiazepin-Rezeptoren entfalten.